(19) Europäisches Patentamt
European Patent Office
Office européen des brevets

(11) Veröffentlichungsnummer: **0 244 739 B1**

(12) # EUROPÄISCHE PATENTSCHRIFT

(45) Veröffentlichungstag der Patentschrift: **27.07.94**

(51) Int. Cl.$^{5}$: **C07D 207/06**, C07D 211/14, A01N 43/36, A01N 43/40

(21) Anmeldenummer: **87106161.0**

(22) Anmeldetag: **28.04.87**

(54) **N-substituierte Pyrrolidin- und Piperidinderivate und deren Salze.**

(30) Priorität: **02.05.86 DE 3614907**

(43) Veröffentlichungstag der Anmeldung:
**11.11.87 Patentblatt 87/46**

(45) Bekanntmachung des Hinweises auf die Patenterteilung:
**27.07.94 Patentblatt 94/30**

(84) Benannte Vertragsstaaten:
**AT BE CH DE ES FR GB GR IT LI NL SE**

(56) Entgegenhaltungen:
**EP-A- 0 000 333**
**EP-A- 0 007 067**
**EP-A- 0 182 224**
**DE-A- 2 801 195**

(73) Patentinhaber: **BASF Aktiengesellschaft**
**Carl-Bosch-Strasse 38**
**D-67063 Ludwigshafen(DE)**

(72) Erfinder: **Buschmann, Ernst, Dr.**
**Georg-Ludwig-Krebs-Strasse 10**
**D-6700 Ludwigshafen(DE)**
Erfinder: **Himmele, Walter, Dr.**
**Eichenweg 14**
**D-6909 Walldorf(DE)**
Erfinder: **Ammermann, Eberhard, Dr.**
**Sachsenstrasse 3**
**D-6700 Ludwigshafen(DE)**
Erfinder: **Pommer, Ernst-Heirich, Dr.**
**Berliner Platz 7**
**D-6703 Limburgerhof(DE)**

EP 0 244 739 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue N-substituierte Pyrrolidin- und Piperidinderivate der allgemeinen Formel I

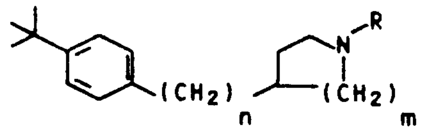

(I)

in der der Substituent und die Indices folgende Bedeutung haben:

- R $C_1$-$C_{20}$-Alkyl, $C_2$-$C_{20}$-Alkoxyalkyl, $C_2$-$C_{20}$-Hydroxyalkyl, $C_3$-$C_{12}$-Cycloalkyl, $C_4$-$C_{20}$-Alkyl-cycloalkyl, durch Hydroxy substituiertes $C_4$-$C_{20}$-Alkyl-cycloalkyl, $C_4$-$C_{20}$-Cycloalkyl-alkyl, Aryl, Halogenaryl, $C_7$-$C_{20}$-Halogenaryl-alkyl, $C_8$-$C_{20}$-Trifluormethylaryl-alkyl oder $C_7$-$C_{20}$-Aryloxy-alkyl,
- m 1 oder 2,
- n 0 oder 1

und deren pflanzenphysiologisch verträgliche Salze.

Außerdem betrifft die Erfindung die Herstellung der Verbindungen der allgemeinen Formel I, die Verwendung dieser Verbindungen als Fungizide, sowie Fungizide, welche diese Verbindungen enthalten und Verfahren zur Bekämpfung von Pilzen.

Phenyl- und Benzylpyrrolidine und -piperidine, die im aromatischen Rest auch substituiert sein können, sind als Antidepressiva (DE-OS 28 01 195, US-A 3 632 767) und als Muskelrelaxantien (DE-OS 20 17 255, DE-OS 20 17 256) bekannt.

Weiterhin ist die Verbindung N-[3-p-tert.-Butyl-phenyl-2-methyl-1-propyl]-cis-2,6-dimethylmorpholin (Fenpropimorph) I' aus der DE-OS 26 56 747

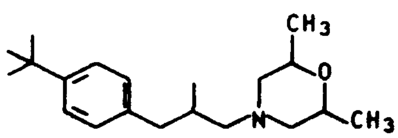

(I')

als handelsübliches Fungizid bekannt.

Der Erfindung lag die Aufgabe zugrunde, neue wirksame Fungizide bereitzustellen.

Demgemäß wurden die eingangs definierten neuen N-substituierten Pyrrolidin- und Piperidinderivate I und deren Salze gefunden. Weiterhin wurde gefunden, daß die Verbindungen I und deren pflanzenphysiologisch verträglichen Salze sich hervorragend als Fungizide eignen.

Außerdem wurden Verfahren zur Herstellung der Verbindungen I und deren Salzen gefunden.

Die Verbindungen des Typs I sind nach folgenden Methoden erhältlich:
Die Verbindungen I können, wie aus folgendem Schema hervorgeht, durch tert.-Butylierung des Aromaten und anschließender N-Substitution des sekundären Amins oder durch Umkehr der Reaktionsfolge aus derselben Ausgangsverbindung IV hergestellt werden.

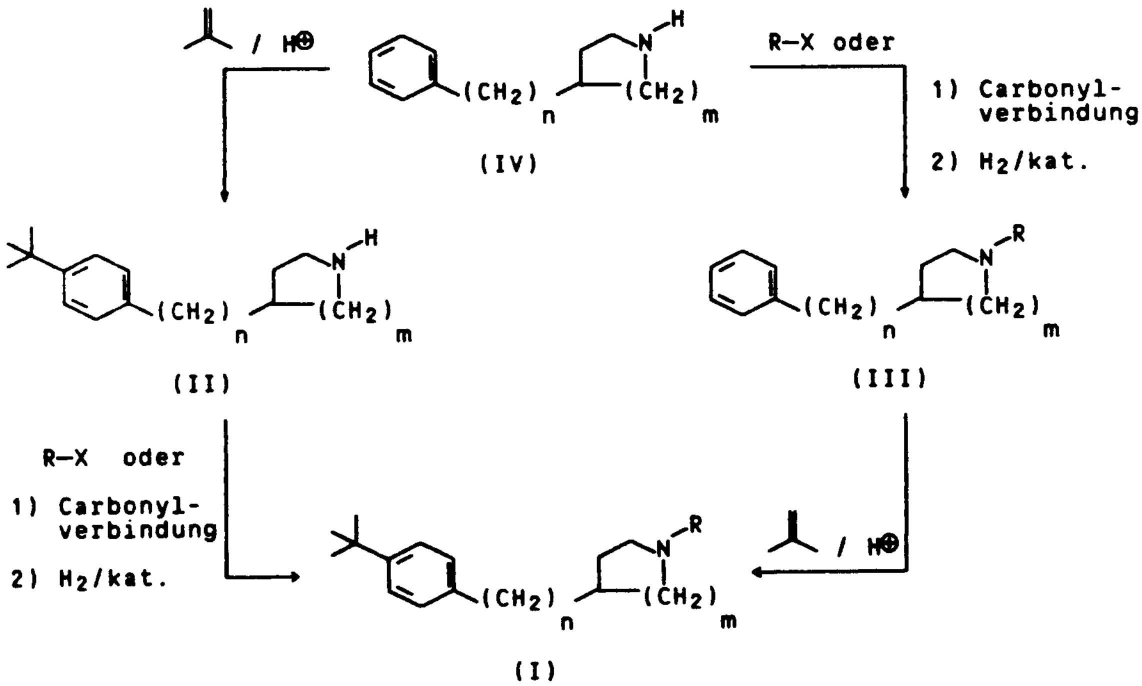

(IV)
(II)
(III)
(I)

a) Einführung des Restes R

$a_1$) Mit Halogeniden R-X

Die Substitution am Stickstoff der Arylamine II und IV kann in an sich bekannter Weise gegebenenfalls in Gegenwart eines Lösungs- oder Verdünnungsmittels und/oder einer Base und/oder eines Reaktionsbeschleunigers durchgeführt werden. Zur Reaktionsbeschleunigung verwendet man insbesondere Jodide, beispielsweise Kalium- oder Natriumjodid. Als Lösungs- oder Verdünnungsmittel werden die hierfür üblichen eingesetzt wie Chlorkohlenwaserstoffe, z.B. Tetrachlorethylen, Tetrachlorethan, Methylenchlorid, Chloroform, Tetrachlorkohlenstoff, Trichlorethan und Chlorbenzol; Ether, z.B. Di-n-butylether, Di-isopropylether, Tetrahydrofuran, Ethylenglykoldimethylether und Dioxan; Nitrokohlenwasserstoffe, z.B. Nitromethan und Nitrobenzol; Ketone, z.B. Cyclopentanon, Cyclohexanon und Aceton oder Amide, z.B. Formamid, Dimethylformamid und Dimethylacetamid. Als Basen können alle üblichen anorganischen und organischen Säurebindemittel verwendet werden. Hierzu gehören vorzugsweise tertiäre Amine, Alkali- und Erdalkaliverbindungen sowie entsprechende Gemische.

$a_2$) Mit Carbonylverbindungen

Die Enaminbildung bei der Umsetzung der Amine II und IV mit einer Carbonylverbindung

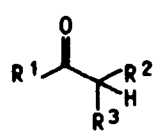

kann in an sich bekannter Weise säurekatalysiert unter Entfernung des Reaktionswassers oder z.B. durch Zusatz von wasserbindenden Mitteln wie z.B. Natrium-, Magnesiumsulfat, Molekularsieb oder Titantetrachlorid erfolgen. Geeignete Lösungsmittel sind beispielsweise Kohlenwasserstoffe, wie Toluol, Xylol und Ligroin; Chlorkohlenwasserstoffe, wie Methylenchlorid, Chloroform und Chlorbenzol oder Ether, wie Diethylether, Diisopropylether, Tetrahydrofuran und Dioxan. Das so erhaltene Enamin läßt sich katalytisch zum Amin hydrieren, z.B. mit Platinoxid (Rylander; Catalytic Hydrogenation over Platinium Metals, Seite 176, Academic Press, New York 1967).

b) Einführung der tert.-Butylgruppe in den Aromaten

Arylamine des Typs III und IV lassen sich mit Isobutylen säurekatalysiert in an sich bekannter Weise zu den p-tert.-Butyl-substituierten Phenylverbindungen umsetzen. Geeignete Lösungsmittel sind besonders Halogenkohlenwasserstoffe, wie Methylenchlorid, Chloroform, Tetrachlorkohlenstoff, Tetrachlorethan, Trrichlorethan und Chlorbenzol sowie die hierfür üblichen Lösungsmittel. Man verwendet Mineralsäuren wie

beispielsweise Schwefelsäure und führt die Reaktion unter Eiskühlung z.B. bei -5 bis +10 °C durch. Das Produkt kann sowohl als Salz, beispielsweise als Hydrogensulfat, oder als freies Amin gewonnen werden.

c) Herstellung von Salzen der N-substituierten Pyrrolidin- und Piperidinverbindungen

Das cyclische tertiäre Amin I kann in einem inerten Lösungsmittel aufgelöst werden und die Salzbildung in an sich bekannter Weise bei gasförmigen Säuren z.B. HCl-Gas durch Einleiten in die Lösung, bei flüssigen Säuren wie Phosphorsäure oder Schwefelsäure durch Zutropfen in equimolaren Mengen, sowie bei festen Säuren durch Lösen einer equimolaren Menge in einem inerten Lösungsmittel und anschließendem Zutropfen erfolgen. Die Reaktion läßt sich bei Raumtemperatur, gegebenenfalls unter Eiskühlung durchführen.

Unter den pflanzenverträglichen Salzen der cyclischen tertiären Amine können beispielsweise diejenigen mit HCl, HBr, HJ, $H_2SO_4$, $H_3PO_4$, $HNO_3$, $HCO_2H$, $CH_3CO_2H$, $C_2H_5CO_2H$ oder $C_{12}H_{25}$-$C_6H_5$-$SO_3H$ gebildet werden.

Die neuen N-substituierten Pyrrolidin- und Piperidinderivate der Formel I und deren pflanzenphysiologisch verträglichen Salze enthalten gegebenenfalls chirale Zentren. Sie werden im allgemeinen als Racemate oder gegebenenfalls als Diastereomerengemische erhalten. Diastereomerenreine Verbindungen lassen sich bei einigen der neuen Verbindungen beispielsweise durch Destillation, Säulenchromatographie oder aufgrund von Löslichkeitsunterschieden isolieren. Enantiomerenreine Verbindungen lassen sich z.B. durch Racematspaltung mit einem chiralen Hilfsreagenz nach bekannten Methoden beispielsweise über diastereomere Salze erhalten. Für die Anwendung der neuen N-substituierten Pyrrolidin- und Piperidinderivate I und deren pflanzenphysiologisch verträglichen Salze als Fungizide sind sowohl die Diastereomeren bzw. die Enantiomeren als auch deren bei der Synthese anfallenden Stereoisomerengemisch geeignet.

Der Rest R in den Verbindungen I bedeutet:

- $C_1$-$C_{20}$-Alkyl, bevorzugt $C_1$-$C_{10}$-Alkyl, besonders bevorzugt $C_4$-$C_9$-Alkyl, wie n-Butyl, iso-Butyl, tert.-Butyl, n-Pentyl, iso-Pentyl, n-Hexyl, n-Octyl, n-Nonyl, 4,4-Dimethylpentyl und 3,5,5-Trimethylhexyl, 3,3-Dimethyl-n-butyl, 1,1,2-Trimethyl-n-propyl und 1,1,3,3-Tetramethyl-n-butyl;
- $C_2$-$C_{20}$-Alkoxyalkyl, bevorzugt $C_2$-$C_8$-Alkoxyalkyl, wie Methoxymethyl, Methoxyethyl, Methoxy-n-propyl, Methoxybutyl, Methoxyhexyl, Ethoxymethyl, Ethoxyethyl, Ethoxybutyl, Propoxymethyl, Propoxyethyl, Butoxymethyl, Butoxypropyl;
- $C_2$-$C_{20}$-Hydroxyalkyl, bevorzugt $C_2$-$C_8$-Hydroxyalkyl, wie Hydroxyethyl, Hydroxypropyl, Hydroxybutyl, Hydroxyhexyl, Hydroxyoctyl;
- $C_3$-$C_{12}$-Cycloalkyl, bevorzugt $C_5$-$C_8$-Cycloalkyl, besonders bevorzugt Cyclopentyl und Cyclohexyl;
- $C_4$-$C_{20}$-Alkyl-cycloalkyl, bevorzugt $C_4$-$C_{12}$-Alkyl-cycloalkyl, besonders bevorzugt $C_8$-$C_{10}$-Alkyl-cycloalkyl, wie 3-Methyl-5-n-propyl-cyclohexyl, 3,5-Diethyl-cyclohexyl, 4-(tert.-Butyl)-cyclohexyl, 1-Ethyl-cyclohexyl, 3,3-Dimethyl-cyclohexyl und 3,3,5-Trimethyl-cyclohexyl;
- durch Hydroxy substituiertes $C_4$-$C_{20}$-Alkyl-cycloalkyl, bevorzugt durch Hydroxy substituiertes $C_4$-$C_{12}$-Alkyl-cycloalkyl, besonders bevorzugt durch Hydroxy substituiertes $C_6$-$C_{10}$-Alkyl-cycloalkyl, wie 3,3-Dimethyl-4-hydroxy-cyclohexyl;
- $C_4$-$C_{20}$-Cycloalkyl-alkyl, bevorzugt $C_4$-$C_{12}$-Cycloalkyl-alkyl, besonders bevorzugt $C_6$-$C_8$-Cycloalkyl-alkyl, wie Cyclopentyl-methyl, Cyclopentyl-ethyl, Cyclohexyl-methyl, Cyclohexyl-ethyl und 2-Cyclohexyl-ethyl;
- Aryl, bevorzugt Phenyl und Naphthyl;
- Halogenaryl, bevorzugt Fluor- und Chloraryl, besonders bevorzugt Chloraryl, wie 4-Chlorphenyl, 2,4-Dichlorphenyl und 3,5-Dichlorphenyl;
- $C_7$-$C_{20}$-Halogenaryl-alkyl, bevorzugt $C_7$-$C_{14}$-Halogenaryl-alkyl, besonders bevorzugt $C_7$-$C_{10}$-Fluor- und Chloraryl-alkyl, wie 4-Fluorbenzyl, 4-Chlorbenzyl, 4-Chlorphenylethyl, 4-Chlorphenylpropyl, 4-Chlorphenylbutyl;
- $C_8$-$C_{20}$-Trifluormethylaryl-alkyl, bevorzugt $C_8$-$C_{14}$-Trifluormethylaryl-alkyl, besonders bevorzugt $C_8$-$C_{10}$-Trifluormethylaryl-alkyl, wie 4-Trifluormethylbenzyl und 4-Trifluormethylphenyl-ethyl;
- $C_7$-$C_{20}$-Aryloxy-alkyl, bevorzugt $C_7$-$C_{14}$-Aryloxy-alkyl, wie Phenoxymethyl, Phenoxyethyl, Phenoxypropyl, Phenoxybutyl, Phenoxyhexyl, Phenoxyoctyl.

Bei den Indizes sind für m die Zahl 2 besonders bevorzugt und für n die Zahl 0.

Als pflanzenphysiologisch verträgliche Salze der Verbindungen I werden die Hydrochloride besonders bevorzugt.

Beispiele für geeignete Verbindungen I sind:

| Verb. Nr. | Verbindung I |
|---|---|
| 1 | N-Methyl-4-(p-tert.-butylphenyl)-piperidin |
| 2 | N-Isopropyl-4-(p-tert.-butylphenyl)-piperidin |
| 3 | N-Isobutyl-4-(p-tert.-butylphenyl)-piperidin |
| 4 | N-Isopentyl-4-(p-tert.-butylphenyl)-piperidin |
| 5 | N-n-Hexyl-4-(p-tert.-butylphenyl)-piperidin |
| 6 | N-(4,4-Dimethylpentyl)-4-(p-tert.-butylphenyl)-piperidin |
| 7 | N-(3,5,5-Trimethylhexyl)-4-(p-tert.-butylphenyl)-piperidin |
| 8 | N-Cyclopentyl-4-(p-tert.-butylphenyl)-piperidin |
| 9 | N-Cyclohexyl-4-(p-tert.-butylphenyl)-piperidin |
| 10 | N-(3-Methyl-5-n-propyl-cyclohexyl)-4-(p-tert.-butylphenyl)-piperidin |
| 11 | N-(4-tert.-Butyl-cyclohexyl)-4-(p-tert.-butylphenyl)-piperidin |
| 12 | N-Cyclooctyl-4-(p-tert.-butylphenyl)-piperidin |
| 13 | N-(Cyclohexyl-methyl)-4-(p-tert.-butylphenyl)-piperidin |
| 14 | N-(2-Cyclohexyl-ethyl)-4-(p-tert.-butylphenyl)-piperidin |
| 15 | N-(3-Cyclohexyl-n-propyl)-4-(p-tert.-butylphenyl)-piperidin |
| 16 | N-(2-Cyclohexyl-n-propyl)-4-(p-tert.-butylphenyl)-piperidin |
| 17 | N-(2-Hydroxyethyl)-4-(p-tert.-butylphenyl)-piperidin |
| 18 | N-(2-Methoxyethyl)-4-(p-tert.-butylphenyl)-piperidin |
| 19 | N-(2-Methoxy-n-propyl)-4-(p-tert.-butylphenyl)-piperidin |
| 20 | N-(4-Chlorbenzyl)-4-(p-tert.-butylphenyl)-piperidin |
| 21 | N-(4-Trifluormethyl-benzyl)-4-(p-tert.-butylphenyl)-piperidin |
| 22 | N-(2-Phenoxy-ethyl)-4-(p-tert.-butylphenyl)-piperidin |
| 23 | N-(Cyclohexyl-methyl)-4-(p-tert.-butylphenyl)-pyrrolidin |
| 24 | N-(2-Cyclohexyl-ethyl)-4-(p-tert.-butyl-benzyl)-piperidin |
| 25 | N-(4-Chlorbenzyl)-4-(p-tert.-butyl-benzyl)-piperidin |
| 26 | N-(Cyclohexyl-methyl)-4-(p-tert.-butyl-phenyl)-pyrrolidin |
| 27 | N-(2-Cyclohexyl-ethyl)-4-(p-tert.-butyl-phenyl)-pyrrolidin |
| 28 | N-Cyclohexyl-4-(p-tert.-butyl-phenyl)-pyrrolidin |
| 29 | N-Cyclopentyl-4-(p-tert.-butyl-phenyl)-pyrrolidin |

| Verb. Nr. | Verbindung I |
|---|---|
| 30 | N-Cyclohexyl-4-(p-tert.-butyl-benzyl)-pyrrolidin |
| 31 | N-Cyclopentyl-4-(p-tert.-butyl-benzyl)-pyrrolidin |
| 32 | N-Isobutyl-4-(p-tert.-butylphenyl)-piperidinium-hydrochlorid |
| 33 | N-n-Hexyl-4-(p-tert.-butylphenyl)-piperidinium-hydrochlorid |
| 34 | N-Cyclohexyl-4-(p-tert.-butylphenyl)-piperidinium-hydrochlorid |
| 35 | N-Cyclopentyl-4-(p-tert.-butylphenyl)-piperidinium-hydrochlorid |
| 36 | N-(3,3-Dimethyl-n-butyl)-4-(p-tert.-butylphenyl)-piperidin |
| 37 | N-(3,3-Dimethyl-n-butyl)-4-(p-tert.-butylbenzyl)-piperidin |
| 38 | N-(3,3-Dimethyl-cyclohexyl)-4-(p-tert.-butylphenyl)-piperidin |
| 39 | N-(3,3-Dimethyl-cyclohexyl)-4-(p-tert.-butylbenzyl)-piperidin |
| 40 | N-(3,3,5-Trimethyl-cyclohexyl)-4-(p-tert.-butylphenyl)-piperidin |
| 41 | N-(3,3,5-Trimethyl-cyclohexyl)-4-(p-tert.-butylbenzyl)-piperidin |
| 42 | N-tert.-Butyl-4-(p-tert.-butylphenyl)-piperidin |
| 43 | N-(1-Ethyl-cyclohexyl-4-(p-tert.-butylphenyl)-piperidin |
| 44 | N-(1,1,2-Trimethyl-n-propyl)-4-(p-tert.-butylphenyl)-piperidin |
| 45 | N-(1,1,3,3-Tetramethyl-n-butyl)-4-(p-tert.-butylphenyl)-piperidin |
| 46 | N-(4-Hydroxy-3,3,5-trimethyl-cyclohexyl)-4-(p-tert.-butylphenyl)-piperidin |
| 47 | N-(4-Hydroxy-3,3,5-trimethyl-cyclohexyl)-4-(p-tert.-butylbenzyl)-piperidin |
| 48 | N-(3,3-Dimethyl-4-hydroxy-cyclohexyl)-4-(p-tert.-butylphenyl)-piperidin |
| 49 | N-(3,3-Dimethyl-4-hydroxy-cyclohexyl)-4-(p-tert.-butylbenzyl)-piperidin |
| 50 | N-(4-Hydroxy-cyclohexyl)-4-(p-tert.-butylphenyl)-piperidin |
| 51 | N-(4-Hydroxy-cyclohexyl)-4-(p-tert.-butylbenzyl)-piperidin |
| 52 | N-(p-Chlorbenzyl)-4-(p-tert.-butylbenzyl)-piperidin |

Die N-substituierten Pyrrolidin- und Piperidinderivate der Formel I und deren pflanzenphysiologisch verträglichen Salze bzw. die sie enthaltenden fungiziden Mittel sowie die daraus hergestellten gebrauchsfertigen Zubereitungen können beispielsweise in Form von direkt versprühbaren Lösungen, Emulsionen, Suspensionen, Stäuben, Pulvern, Pasten oder Granulaten in an sich bekannter Weise, z.B. durch Versprühen, Vernebeln, Verstäuben, Verstreuen, Beizen oder Gießen angewendet werden, Die Anwendungsformen richten sich ganz nach den Verwendungszwecken. Sie sollen in jedem Fall eine feine und gleichmäßige Verteilung der wirksamen Substanz gewährleisten. Die Formulierungen werden in an sich bekannter Weise z.B. durch Verstrecken des Wirkstoffes mit Lösungsmitteln und/oder Trägerstoffen, gegebenenfalls unter Verwendung von Emulgier- oder Dispergiermitteln hergestellt. Als Lösungsmittel werden beispielsweise Aromaten wie Benzol, Toluol oder die Xylole, chlorierte Aromaten wie Chlor- und Brombenzol und deren Derivate, Paraffine wie diverse Erdölfraktionen, Alkohole wie Methanol, Ethanol, n- und iso-Propanol oder n-, iso- und tert.-Butanol, Ketone wie Aceton, Cyclopentanon oder Cyclohexanon, Amine wie Ethanolamin, Dimethylamin, Triethylamin oder Dimethylformamid, oder Wasser verwendet. Bei der Benutzung von Wasser als Verdünnungsmittel werden Lösungsvermittler gebraucht. Als solche dienen organische Lösungsmittel, die mit Wasser mischbar oder gegebenenfalls nur teilweise mischbar sind wie Aceton, Methanol, Ethanol, Ethanolamin, Dimethylamin oder Triethylamin. Als Trägerstoffe eignen sich natürliche Gesteinsmehle wie Kaolin, Kreide, Talkum oder Tonerde, oder synthetische Gesteinsmehle wie hochdisperse Kieselsäure oder Silikate. Als Emulgatoren werden nichtionogene oder anionische wie z.B. Polyoxyethylen-Fettalkohol-Ether, Alkyl- oder Arylsulfonate und als Dispergiermittel beispielsweise Lignin, Sulfitablaugen oder Methylcellulose verwendet.

Die N-substituierten Pyrrolidin- und Piperidinderivate der Formel I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind z.T. systemisch wirksam und können als Blatt- und/oder Bodenfungizide eingesetzt werden.

Besonders wichtig sind die Verbindungen I für die Bekämpfung einer Vielzahl von Pilzen an verschiedenen Kulturpflanzen und/oder deren Samen, beispielsweise für Weizen, Roggen, Gerste, Hafer, Reis, Mais, Baumwolle, Soja, Kaffee, Zuckerrohr, Erdnüsse, Obst oder Zierpflanzen im Garten- oder Weinbau, sowie für Gemüse, wie Gurken, Bohnen oder Kürbisgewächse.

Die Verbindungen I eignen sich besonders zur Bekämpfung der aufgeführten Pflanzenkrankheiten in/an den entsprechenden Nutzpflanzen.

| Pflanzenkrankheiten | Nutzpflanzen |
|---|---|
| Erysiphe graminis, Puccinia-Arten | Getreide |
| Septoria nodorum | Weizen |
| Helminthosporium teres | Gerste |
| Pseudocercosporella herpotrichoides | Weizen, Gerste |
| Ustilago-Arten | Getreide, Zuckerrohr |
| Pyricularia oryzae | Reis |
| Cercospora arachidicola | Erdnüsse |
| Podosphaera leucotricha, Venturia inaequalis | Äpfel |
| Botrytis cinerea | Erdbeeren, Reben |
| Plasmopara viticola, Uncinula necator | Reben |

| Pflanzenkrankheiten | Nutzpflanzen |
|---|---|
| Alternaria solani, Phytophthora infestans | Kartoffeln, Tomaten |
| Erysiphe cichoracearum, Sphaerotheca fuliginea | Kürbisgewächse |
| Fusarium-, Verticillium-Arten | verschiedene Pflanzen |

Die fungiziden Mittel enthalten im allgemeinen zwischen 0,1 und 95 Gew.%, vorzugsweise zwischen 0,5 und 90 Gew.% Wirkstoff.

Die Aufwandmengen an Wirkstoff betragen je nach Art des gewünschten Effektes zwischen 0,02 und 3 kg Wirkstoff/ha oder darüber. Die Applikation der N-substituierten Pyrrolidin- und Piperidinderivate I erfolgt vor oder nach der Infektion der Pflanzen oder Samen entweder durch Besprühen oder Bestäuben.

Die Verbindungen I können auch im Materialschutz, beispielsweise zur Bekämpfung holzzerstörender Pilze wie Coniophora puteanea oder Polystictus versicolor eingesetzt werden.

Ferner lassen sich diese Verbindungen auch als fungizid wirksame Bestandteile öliger Holzschutzmittel gegen holzverfärbende Pilze, z.B. durch Anstreichen oder Tränken des Holzes, verwenden.

Einige der Verbindungen I eignen sich zur Bekämpfung hautpathogener Pilze, wie Trichophyton mentagrophytes oder Candida albicans.

Die N-substituierten Pyrrolidin- und Piperidinderivate I können auch mit anderen Wirkstoffen, wie z.B. Herbiziden, Insektiziden, Wachstumsregulatoren oder Düngemitteln vermischt und ausgebracht werden.

Herstellungsbeispiele

Beispiele 1 und 3

N-Substitution mit Halogeniden R-X

Eine Lösung von 434 mmol eines cyclischen Amins II, 430 mmol eines Halogenids R-X und 1,3 mol Natriumcarbonat in 400 ml Dimethylformamid wurde 8 Stunden auf 150 °C erhitzt, wonach das Reaktionsgemisch wie üblich aufgearbeitet wurde.

Einzelheiten und Ergebnisse sind Tabelle 1 zu entnehmen.

**Tabelle 1**

| | Verb.Nr. | Verbindung II | | Halogenid | | Verbindung I | |
|---|---|---|---|---|---|---|---|
| Bsp.Nr. | | m | n | R | X | Sdp.[°C]/mbar | Ausbeute [%] |
| 1 | 5 | 2 | 0 | n-Hexyl | Br | 160-170 /1,0 | 82 |
| 2 | 14 | 2 | 0 | 2-Cyclohexylethyl | Br | 185-186 /0,2 | 75 |
| 3 | 36 | 2 | 0 | 3,3-Dimethyl-n-butyl | Cl | 177 /2 | 65 |

Beispiele 4 bis 15

a) Darstellung der Enamine
Eine Lösung von 2 mol eines cyclischen Amins II, 1 mol einer Carbonylverbindung und 1 g Toluolsulfonsäure in 400 ml Toluol wurden am Wasserabscheider erhitzt und wie üblich aufgearbeitet.

b) Hydrierung der Enamine
Eine Lösung von 460 mmol Enamin in 1 l Essigsäure-ethylester wurde in Gegenwart von 10 g 10proz. Pd/C bei 60 bis 70 °C und 100 bar hydriert und wie üblich aufgearbeitet.

Einzelheiten und Ergebnisse sind in Tabelle 2 zusammengestellt.

Tabelle 2

| Bsp.Nr. | Verb.Nr. | Verbindung II n | m | Carbonylverbindung | Verbindung I R | Sdp[°C]/mbar | Ausbeute [%] |
|---|---|---|---|---|---|---|---|
| 4 | 3 | 0 | 2 | Isobutyraldehyd | Isobutyl | 155-160/0,8 | 55 |
| 5 | 4 | 0 | 2 | Isovaleraldehyd | Isopentyl | 165-168/1,0 | 40 |
| 6 | 6 | 0 | 2 | γ,γ-Dimethylvaleraldehyd | 4,4-Dimethylpentyl | 160 /0,3 | 60 |
| 7 | 7 | 0 | 2 | 2,4,4-Trimethylhexanal | 3,5,5-Trimethylhexyl | 182-184/0,3 | 65 |
| 8 | 8 | 0 | 2 | Cyclopentanon | Cyclopentyl | 172-180/1,0 | 35 |
| 9 | 9 | 0 | 2 | Cyclohexanon | Cyclohexyl | 188-194/2,0 | 66 |
| 10 | 10 | 0 | 2 | 3-Methyl-5-n-propylcyclohexanon | 3-Methyl-5-n-propyl-cyclohexyl | 190-192/0,4 | 70 |

Tabelle 2

| Bsp.Nr. | Verb.Nr. | Verbindung II n | m | Carbonylverbindung | Verbindung I R | Sdp[°C]/mbar | Ausbeute [%] |
|---|---|---|---|---|---|---|---|
| 11 | 13 | [illegible] | 2 | Cyclohexancarbaldehyd | Cyclohexylmethyl | 165-170/0,3 | 65 |
| 12 | 23 | [illegible] | 1 | Cyclohexancarbaldehyd | Cyclohexylmethyl | 152-156/0,2 | 70 |
| 13 | 24 | [illegible] | 2 | Cyclohexancarbaldehyd | Cyclohexylmethyl | 174-178/0,3 | 48 |
| 14 | 38 | 0 | 2 | 3,3-Dimethylcyclohexanon | 3,3-Dimethylcyclohexyl | * | 36 |
| 15 | 40 | 0 | 2 | 3,3,5-Trimethylcyclohexanon | 3,3,5-Trimethylcyclohexyl | 130-138/1 | 34 |

*) Fp. [°C]: 119-121

Beispiele 16 bis 22

Einführung der tert.-Butylgruppe in den Aromaten

Zu einer Lösung von 275 mmol eines cyclischen Amins in 500 ml Methylenchlorid wurde unter Eiskühlung bei 5 bis 10 °C 1,4 mol konz. Schwefelsäure zugetropft und anschließend bei 10 bis 15 °C 375 mmol Isobutylen eingegast. Nach einstündigem Rühren bei Raumtemperatur wurde wie üblich aufgearbeitet.

Einzelheiten und Ergebnisse sind Tabelle 3 zu entnehmen.

Tabelle 3

| Bsp.Nr. | Verb.Nr. | Verbindungen III n | m | R | Verbindungen I Sdp.[°C]/mbar | Fp [°C] | Ausbeute [%] |
|---|---|---|---|---|---|---|---|
| 16 | 5 | 0 | 2 | n-Hexyl | 160-170 /1,0 | | 45 |
| 17 | 8 | 0 | 2 | Cyclopentyl | 172-180 /1,0 | | 52 |
| 18 | 9 | 0 | 2 | Cyclohexyl | 188-194 /2,0 | | 62 |
| 19 | 42 | 0 | 2 | tert.-Butyl | | 74 | 65 |
| 20 | 43 | 0 | 2 | 1-Ethyl-cyclohexyl | | 86-88 | 52 |
| 21 | 44 | 0 | 2 | 1,1,2-Tri-methyl-n-propyl | | 108 | 64 |
| 22 | 45 | 0 | 2 | 1,1,3,3-Tetra-methyl-n-butyl | | 86-88 | 36 |

Beispiel 23

Herstellung von N-Isobutyl-4-(p-tert.-butylphenyl)-piperidinium-hydrochlorid (Verbindung 32)

In eine Lösung von 73 mmol N-Isobutyl-4-(p-tert.-butylphenyl)-piperidin wurde HCl-Gas bei Raumtemperatur eingeleitet. Die übliche Aufarbeitung lieferte 67 % der Verbindung 32; Smp. 246°C (unter Zersetzung).

Anwendungsbeispiele

Beispiel A

Wirksamkeit gegen Gurkenmehltau

Blätter von in Töpfen gewachsenen Gurkenkeimlingen der Sorte "Chinesische Schlange" wurden im Zweiblattstadium mit einer Sporensuspension des Gurkenmehltaus (Erysiphe cichoracearum und Sphaerotheca fuliginea) besprüht. Nach etwa 20 Stunden wurden die Versuchspflanzen mit einer wäßrigen Spritzbrühe, die 80 Gew.% Wirkstoff und 20 Gew.% Emulgiermittel in der Trockensubstanz enthielt, bis zur Tropfnässe besprüht. Nach dem Antrocknen des Spritzbelages wurden sie anschließend im Gewächshaus bei Temperaturen zwischen 20 und 22°C und 70 bis 80 % relativer Luftfeuchtigkeit aufgestellt. Zur Beurteilung der Wirksamkeit der neuen Stoffe wurde das Ausmaß der Pilzentwicklung nach 21 Tagen ermittelt.

Das Ergebnis des Versuches zeigte, daß die Verbindungen 4, 5, 6, 7, 8, 9, 13, 14, 23, 24 und 32 der Beispiele 1, 2, 4, 5, 6, 7, 8, 10, 11, 12, 13, 14, 15 und 16 bei der Anwendung als 0.025 %ige Spritzbrühe eine bessere fungizide Wirkung (mit 97 bis 100 %) zeigten als die Vergleichsverbindung Fenpropimorph (mit 70 %).

Beispiel B

Wirksamkeit gegen Plasmopara viticola

Blätter von Topfreben der Sorte "Muller-Thurgau" wurden mit einer wäßrigen Spritzbrühe, die 80 Gew.% Wirkstoff und 20 Gew.% Emulgiermittel in der Trockensubstanz enthielt, besprüht. Um die

Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des Sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis des Versuches zeigte, daß die Verbindungen 4, 5, 6, 7, 8, 9, 10, 13, 14, 23, 24 und 32 der Beispiele 1, 2, 4, 5, 6, 7, 9, 10, 11, 13, 13, 14 und 16 bei der Anwendung als 0,05 %ige Spritzbrühe eine bessere fungizide Wirkung (mit 90 bis 100 %) zeigten als die Vergleichsverbindung Fenpropimorph (mit 50 %).

Beispiel C

Wirksamkeit gegen Septoria nodorum

Die Blätter von in Töpfen gewachsenen Weizenkeimlingen der Sorte "Jubilar" wurden mit einer wäßrigen Spritzbrühe, die 80 Gew.% Wirkstoff und 20 Gew.% Emulgiermittel in der Trockensubstanz enthielt, bis zur Tropfnässe besprüht. Am folgenden Tag wurden die angetrockneten Pflanzen mit einer wäßrigen Sporensuspension von Septoria nodorum infiziert und dann für 7 Tage bei 17 bis 19 °C und 95 %iger relativer Luftfeuchtigkeit weiter kultiviert. Das Ausmaß des Pilzbefalls wurde dann visuell eimittelt.

Das Ergebnis des Versuches zeigte, daß die Verbindungen 3, 4, 8, 13 und 14 der Beispiele 2, 4, 7, 10 und 14 bei der Anwendung als 0,05 %ige Spritzbrühe eine bessere fungizide Wirkung (mit 97 bis 100 %) zeigen als die Vergleichsverbindung Fenpropimorph (mit 90 %).

## Patentansprüche

**Patentansprüche für folgende Vertragsstaaten : BE, CH, DE, FR, GB, IT, LI, NL, SE**

**1.** N-substituierte Pyrrolidin- und Piperidinderivate der allgemeinen Formel I

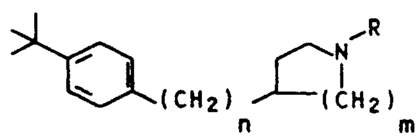

(I)

in der der Substituent und die Indices folgende Bedeutung haben:

R $C_1$-$C_{20}$-Alkyl, $C_2$-$C_{20}$-Alkoxyalkyl, $C_2$-$C_{20}$-Hydroxyalkyl, $C_3$-$C_{12}$-Cycloalkyl, $C_4$-$C_{20}$-Alkyl-cycloalkyl, durch Hydroxy substituiertes $C_4$-$C_{20}$-Alkyl-cycloalkyl, $C_4$-$C_{20}$-Cycloalkyl-alkyl, Aryl, Halogenaryl, $C_7$-$C_{20}$-Halogenaryl-alkyl, $C_8$-$C_{20}$-Trifluormethylaryl-alkyl oder $C_7$-$C_{20}$-Aryloxy-alkyl,

m 1 oder 2,

n 0 oder 1

und deren pflanzenphysiologisch verträgliche Salze.

**2.** N-substituierte Pyrrolidin- und Piperidinderivate der Formel I nach Anspruch 1, in der der Substituent und die Indices folgende Bedeutung haben:

R $C_1$-$C_{10}$-Alkyl, $C_2$-$C_8$-Alkoxyalkyl, $C_2$-$C_8$-Hydroxyalkyl, $C_5$-$C_8$-Cycloalkyl, $C_4$-$C_{12}$-Alkyl-cycloalkyl, durch Hydroxy substituiertes $C_4$-$C_{12}$-Alkyl-cycloalkyl, $C_4$-$C_{12}$-Cycloalkyl-alkyl, Phenyl, Naphthyl, Fluor- und Chloraryl, $C_7$-$C_{14}$-Halogenaryl-alkyl, $C_8$-$C_{14}$-Trifluormethylaryl-alkyl und $C_7$-$C_{14}$-Aryloxy-alkyl,

m 1 oder 2,

n 0 oder 1

und deren pflanzenphysiologisch verträglichen Salze.

**3.** N-substituierte Pyrrolidin- und Piperidinderivate der Formel I nach Anspruch 1, in der der Substituent und die Indices folgende Bedeutung haben:

R $C_4$-$C_9$-Alkyl, Cyclopentyl, Cyclohexvl, $C_8$-$C_{10}$-Alkyl-cycloalkyl, durch Hydroxy substituiertes

$C_6$-$C_{10}$-Alkyl-cycloalkyl, $C_6$-$C_8$-Cycloalkyl-alkyl, Chloraryl, $C_7$-$C_{10}$-Fluor- und Chloraryl-alkyl und $C_8$-$C_{10}$-Trifluormethylaryl-alkyl,

m 2,

n 0

und deren Hydrochloride.

**4.** Verfahren zur Herstellung der Verbindungen I, dadurch gekennzeichnet, daß man

a) die Verbindungen II

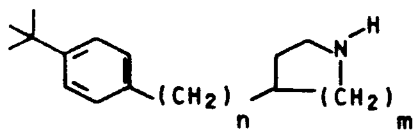

(II)

in an sich bekannter Weise

$a_1$) mit einem Halogenid der Formel R-X, in der X Chlor, Brom oder Jod bedeutet, oder

$a_2$) mit einer Carbonylverbindung

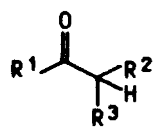

wobei die Reste $R^1$ bis $R^3$ so definiert sind, daß der Rest

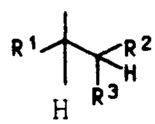

dem Rest R entspricht, umsetzt und anschließend mit Wasserstoff in Gegenwart eines Edelmetallkatalysators das entstandene Enamin hydriert, oder

b) die Verbindungen III

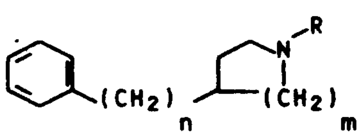

(III)

säurekatalysiert mit Isobutylen umsetzt.

**5.** Fungizid, enthaltend eine Verbindung I oder eines ihrer pflanzenphysiologisch verträglichen Salze neben hierfür üblichen Trägerstoffen.

**6.** Verwendung der Verbindungen I und deren pflanzenphysiologisch verträgliche Salze als Fungizide.

**7.** Verfahren zur Bekämpfung von Pilzen, dadurch gekennzeichnet, daß man die Pilze oder die vom Pilzbefall bedrohten Materialien, Pflanzen, Saatgüter oder Böden mit einem Fungizid gemäß Anspruch 5 behandelt.

**Patentansprüche für folgende Vertragstaaten : AT, ES**

1. Verfahren zur Herstellung von N-substituierten Pyrrolidin- und Piperidinderivaten der allgemeinen Formel I

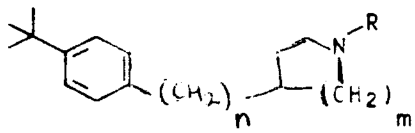

(I)

in der der Substituent und die Indices

R $C_1$-$C_{20}$-Alkyl, $C_2$-$C_{20}$-Alkoxyalkyl, $C_2$-$C_{20}$-Hydroxyalkyl, $C_3$-$C_{12}$-Cycloalkyl, $C_4$-$C_{20}$-Alkyl-cycloalkyl, durch Hydroxy substituiertes $C_4$-$C_{20}$-Alkyl-cycloalkyl, $C_4$-$C_{20}$-Cycloalkyl-alkyl, Aryl, Halogenaryl, $C_7$-$C_{20}$-Halogenaryl-alkyl, $C_8$-$C_{20}$-Trifluormethylaryl-alkyl oder $C_7$-$C_{20}$-Aryloxy-alkyl,

m 1 oder 2,

n 0 oder 1

bedeuten und deren pflanzenphysiologisch verträgliche Salze, dadurch gekennzeichnet, daß man

a) die Verbindungen II

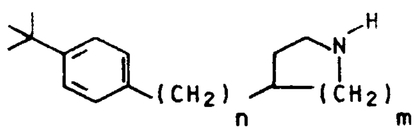

(II)

in an sich bekannter Weise

$a_1$) mit einem Halogenid der Formel R-X, in der X Chlor, Brom oder Jod bedeutet, oder

$a_2$) mit einer Carbonylverbindung

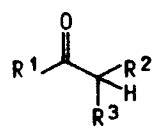

wobei die Reste $R^1$ bis $R^3$ so definiert sind, daß der Rest

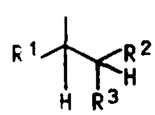

dem Rest R entspricht, umsetzt und anschließend mit Wasserstoff in Gegenwart eines Edelmetallkatalysators das entstandene Enamin hydriert, oder

b) die Verbindungen III

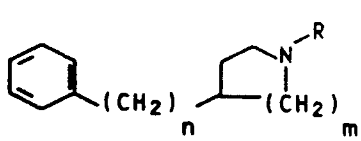

(III)

säurekatalysiert mit Isobutylen umsetzt

und gegebenenfalls in an sich bekannter Weise in deren Salze überführt.

**2.** Fungizid, enthaltend eine Verbindung I oder eines ihrer pflanzenphysiologisch verträglichen Salze neben hierfür üblichen Trägerstoffen.

**3.** Verwendung der Verbindungen I und deren pflanzenphysiologisch verträgliche Salze als Fungizide.

**4.** Verfahren zur Bekämpfung von Pilzen, dadurch gekennzeichnet, daß man die Pilze oder die vom Pilzbefall bedrohten Materialien, Pflanzen, Saatgüter oder Böden mit einem Fungizid gemäß Anspruch 2 behandelt.

## Claims

**Claims for the following Contracting States : BE, CH, DE, FR, GB, IT, LI, SE**

**1.** An N-substituted pyrrolidine or piperidine derivative of the general formula I

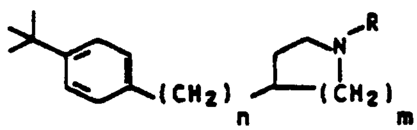

(I)

where the substituent and the indices have the following meanings:

R is $C_1$-$C_{20}$-alkyl, $C_2$-$C_{20}$-alkoxyalkyl, $C_2$-$C_{20}$-hydroxyalkyl, $C_3$-$C_{12}$-cycloalkyl, $C_4$-$C_{20}$-alkylcycloalkyl, $C_4$-$C_{20}$-alkylcycloalkyl substituted by hydroxyl, $C_4$-$C_{20}$-cycloalkylalkyl, aryl, haloaryl, $C_7$-$C_{20}$-haloarylalkyl, $C_8$-$C_{20}$-trifluoromethylarylalkyl or $C_7$-$C_{20}$-aryloxyalkyl,

m is 1 or 2,

n is 0 or 1

or its phytophysiologically tolerable salts.

**2.** An N-substituted pyrrolidine or piperidine derivative of the formula I as claimed in claim 1, where the substituent and the indices have the following meanings:

R is $C_1$-$C_{10}$-alkyl, $C_2$-$C_8$-alkoxyalkyl, $C_2$-$C_8$-hydroxyalkyl, $C_5$-$C_8$-cycloalkyl, $C_4$-$C_{12}$-alkylcycloalkyl, $C_4$-$C_{12}$-alkylcycloalkyl substituted by hydroxyl, $C_4$-$C_{12}$-cycloalkylalkyl, phenyl, naphthyl, fluoro- or chloroaryl, $C_7$-$C_{14}$-haloarylalkyl, $C_8$-$C_{14}$-trifluoromethylarylalkyl or $C_7$-$C_{14}$-aryloxyalkyl,

m is 1 or 2,

n is 0 or 1

or its phytophysiologically tolerable salts.

**3.** An N-substituted pyrrolidine or piperidine derivative of the formula I as claimed in claim 1, where the substituent and the indices have the following meanings:

R is $C_4$-$C_9$-alkyl, cyclopentyl, cyclohexyl, $C_8$-$C_{10}$-alkylcycloalkyl, $C_6$-$C_{10}$-alkylcycloalkyl substituted by hydroxyl, $C_6$-$C_8$-cycloalkylalkyl, chloroaryl, $C_7$-$C_{10}$-fluoro- or chloroarylalkyl or $C_8$-$C_{10}$-trifluoromethylarylalkyl,

m is 2,

n is 0

or its hydrochloride.

**4.** A process for preparing the compounds I, which comprises

a) reacting the compounds II

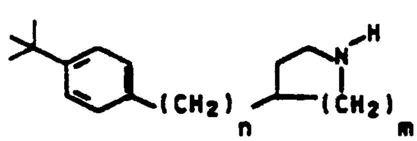

(II)

in a manner known per se

$a_1$) with a halide of the formula R-X, where X is chlorine, bromine or iodine, or

$a_2$) with a carbonyl compound

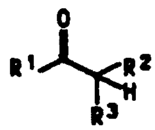

where the radicals $R^1$ to $R^3$ are defined such that the radical

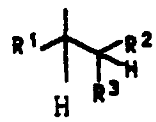

corresponds to the radical R, and then hydrogenating the resultant enamine with hydrogen in the presence of a noble metal catalyst, or

b) reacting the compounds III

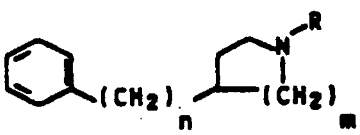

(III)

with isobutylene under acidic catalysis.

**5.** A fungicide, containing a compound I or one of its phytophysiologically tolerable salts in addition to carriers customary for this.

**6.** The use of the compounds I and their phytophysiologically tolerable salts as fungicides.

**7.** A process for controlling fungi, which comprises treating the fungi or the materials, plants, seeds or soils threatened by fungal attack with a fungicide as claimed in claim 5.

**Claims for the following Contracting States : AT, ES**

**1.** A process for preparing N-substituted pyrrolidine and piperidine derivatives of the general formula I

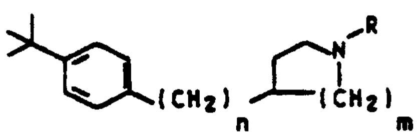

(I)

where the substituent and the indices have the following meanings:

- R is $C_1$-$C_{20}$-alkyl, $C_2$-$C_{20}$-alkoxyalkyl, $C_2$-$C_{20}$-hydroxyalkyl, $C_3$-$C_{12}$-cycloalkyl, $C_4$-$C_{20}$-alkylcycloalkyl, $C_4$-$C_{20}$-alkylcycloalkyl substituted by hydroxyl, $C_4$-$C_{20}$-cycloalkylalkyl, aryl, haloaryl, $C_7$-$C_{20}$-haloarylalkyl, $C_8$-$C_{20}$-trifluoromethylarylalkyl or $C_7$-$C_{20}$-aryloxyalkyl,
- m is 1 or 2,
- n is 0 or 1

and their phytophysiologically tolerable salts, which comprises

a) reacting the compounds II

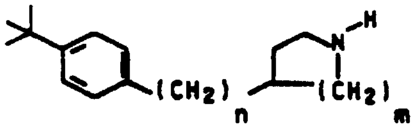

(II)

in a manner known per se

$a_1$) with a halide of the formula R-X, where X is chlorine, bromine or iodine, or

$a_2$) with a carbonyl compound

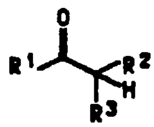

where the radicals $R^1$ to $R^3$ are defined such that the radical

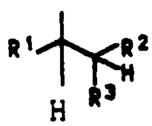

corresponds to the radical R, and then hydrogenating the resultant enamine with hydrogen in the presence of a noble metal catalyst, or

b) reacting the compounds III

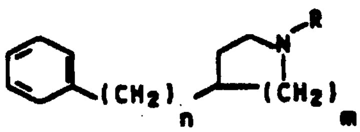

(III)

with isobutylene under acidic catalysis,

and if appropriate converting the products into their salts in a manner known per se.

**2.** A fungicide, containing a compound I or one of its phytophysiologically tolerable salts in addition to carriers customary for this.

**3.** The use of the compounds I and their phytophysiologically tolerable salts as fungicides.

**4.** A process for controlling fungi, which comprises treating the fungi or the materials, plants, seeds or soils threatened by fungal attack with a fungicide as claimed in claim 2.

**Revendications**

**Revendications pour les Etats contractants suivants : BE, CH, DE, FR, GB, IT, LI, SE**

**1.** Dérivés de pyrrolidine et de pipéridine N-substitués de formula générale I

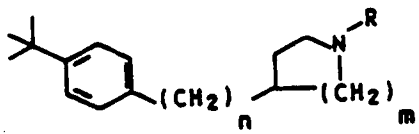

(I)

dans laquelle le substituant et les indices ont la signification suivante :

R alkyle en $C_1$-$C_{20}$, alcoxyalkyle en $C_2$-$C_{20}$, hydroxyalkyle en $C_2$-$C_{20}$, cycloalkyle en $C_3$-$C_{12}$, alkylcycloalkyle en $C_4$-$C_{20}$, alkylcycloalkyle en $C_4$-$C_{20}$ substitué par un groupe hydroxy, cycloalkylalkyle en $C_4$-$C_{20}$, aryle, halogénoaryle, halogénoarylalkyle en $C_7$-$C_{20}$, trifluorométhylarylalkyle en $C_8$-$C_{20}$, ou aryloxyalkyle en $C_7$-$C_{20}$,

m 1 ou 2,

n 0 ou 1

et leurs sels physiologiquement acceptables par les plantes.

**2.** Dérivés de pyrrolidine et de pipéridine N-substitués de formule I selon la revendication 1, dans lesquels le substituant et les indices ont la signification suivante :

R alkyle en $C_1$-$C_{10}$, alcoxyalkyle en $C_2$-$C_8$, hydroxyalkyle en $C_2$-$C_8$, cycloalkyle en $C_5$-$C_8$, alkylcycloalkyle en $C_4$-$C_{12}$, alkylcycloalkyle en $C_4$-$C_{12}$ substitué par un groupe hydroxy, cycloalkylalkyle en $C_4$-$C_{12}$, phényle, naphtyle, fluoro- et chloroaryle, halogénoarylalkyle en $C_7$-$C_{14}$, trifluorométhylarylalkyle en $C_8$-$C_{14}$, et aryloxyalkyle en $C_7$-$C_{14}$,

m 1 ou 2,

n 0 ou 1

et leurs sels physiologiquement acceptables par les plantes.

**3.** Dérivés de pyrrolidine et de pipéridine N-substitués de formule I selon la revendication 1, dans lesquels le substituant et les indices ont la signification suivante :

R alkyle en $C_4$-$C_9$, cyclopentyle, cyclohexyle, alkylcycloalkyle en $C_8$-$C_{10}$, alkylcycloalkyle en $C_6$-$C_{10}$ substitué par un groupe hydroxy, cycloalkylalkyle en $C_6$-$C_8$, chloroaryle, fluoro- et chloroarylalkyle en $C_7$-$C_{10}$ et trifluorométhylarylalkyleen $C_8$-$C_{10}$,

m 2,

n 0

et leurs chlorhydrates.

**4.** Procédé de préparation des composés I, caractérisé en ce que

a) l'on fait réagir les composés II

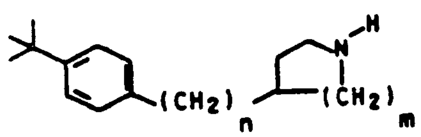

(II)

d'une manière connue en soi

$a_1$) avec un halogénure de formule R-X, dans laquelle X représente un atome de chlore, de brome ou d'iode, ou

$a_2$) avec un composé carbonyle

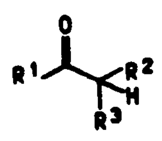

dans lequel les restes $R^1$ à $A^3$ sont définis de telle manière que le reste

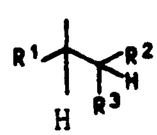

correspond au reste R, puis on traite l'énamine produite par l'hydrogène en présence d'un catalyseur de métal précieux, ou

b) l'on fait réagir les composés III

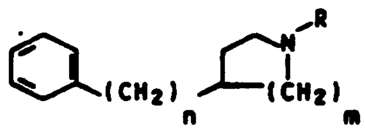

(III)

avec l'isobutylène en catalyse acide.

5. Fongicide, contenant un composé I ou l'un de ses sels physiologiquement acceptables par les plantes ainsi que des matières de support appropriées pour celui-ci.

6. Utilisation des composés I et de leurs sels physiologiquement acceptables par les plantes comme fongicides.

7. Procédé pour combattre les champignons, caractérisé en ce que l'on traite les champignons ou les matières menacées d'une infestation par des champignons, des plantes, des semences ou des sols avec un fongicide selon la revendication 5.

**Revendications pour les Etats contractants suivants : AT, ES**

1. Procédé de préparation de dérivés de pyrrolidine et de pipéridine N-substitués de formule générale I

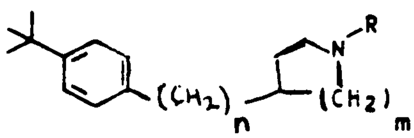

(I)

dans laquelle le substituant et les indices ont la signification suivante :

R alkyle en $C_1$-$C_{20}$, alcoxyalkyle en $C_2$-$C_{20}$, hydroxyalkyle en $C_2$-$C_{20}$, cycloalkyle en $C_3$-$C_{12}$, alkylcycloalkyle en $C_4$-$C_{20}$, alkylcycloalkyle en $C_4$-$C_{20}$ substitué par un groupe hydroxy, cycloalkylalkyle en $C_4$-$C_{20}$, aryle, halogénoaryle, halogénoarylalkyle en $C_7$-$C_{20}$, trifluorométhylarylalkyle en $C_8$-$C_{20}$, ou aryloxyalkyle en $C_7$-$C_{20}$,

m 1 ou 2,

n 0 ou 1

et leurs sels physiologiquement acceptables par les plantes, caractérisé en ce que

a) l'on fait réagir les composés II

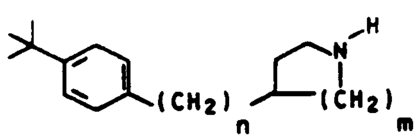

(II)

d'une manière connue en soi

$a_1$) avec un halogénure de formule R-X, dans laquelle X représente un atome de chlore, de brome ou d'iode, ou

$a_2$) avec un composé carbonyle

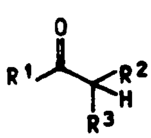

dans lequel les restes $R^1$ à $R^3$ sont définis de telle manière que le reste

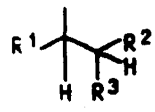

correspond au reste R, puis on traite l'énamine produite par l'hydrogène en présence d'un catalyseur de métal précieux, ou

b) l'on fait réagir les composés III

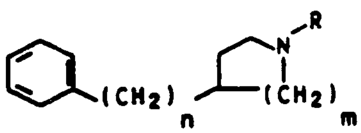

(III)

avec l'isobutylène en catalyse acide

et l'on transforme éventuellement les produits obtenus, d'une manière connue en soi, en leurs sels.

**2.** Fongicide, contenant un composé I ou l'un de ses sels physiologiquement acceptables par les plantes ainsi que des matières de support appropriées pour celui-ci.

**3.** Utilisation des composés I et de leurs sels physiologiquement acceptables par les plantes comme fongicides.

**4.** Procédé pour combattre les champignons, caractérisé en ce que l'on traite les champignons ou les matières menacées d'une infestation par des champignons, des plantes, des semences ou des sols avec un fongicide selon la revendication 2.